# EUROPEAN PATENT SPECIFICATION

(11) **EP 2 701 775 B1**
(45) Date of publication and mention of the grant of the patent: **25.11.2015**
(21) Application number: 12717696.4
(22) Date of filing: 26.04.2012
(51) Int. Cl.: A61M 5/19, A61M 5/24, A61M 5/31

(54) **DISPENSE INTERFACE ASSEMBLY**
AUSGABESCHNITTSTELLENANORDNUNG
ENSEMBLE INTERFACE D'ADMINISTRATION

(30) Priority: 28.04.2011 US 201161480063 P; 08.07.2011 EP 11173280
(43) Date of publication of application: 05.03.2014
(73) Proprietor: Sanofi-Aventis Deutschland GmbH, 65929 Frankfurt am Main (DE)
(72) Inventor: HOLTWICK, Marc, 65926 Frankfurt am Main (DE); DAVIES, James Alexander, Leamington Spa Warwickshire CV32 7XB (GB); BILTON, Simon Lewis, Leamington Spa Warwickshire CV32 5QT (GB); MOORE, David, Elmesthorpe Leicester Leicestershire LE9 7SA (GB); WIMPENNY, Steven, Leamington Spa Warwickshire CV32 6ES (GB); LANGLEY, Christopher Nigel, Leamington Spa Warwickshire CV32 7HH (GB)
(86) International application number: PCT/EP2012/057694
(87) International publication number: WO 2012/146678

(56) References cited:
- EP-A1- 2 335 755
- WO-A1-94/06487
- WO-A2-94/22507
- US-A- 5 378 233

## Description

The present patent application relates to medical devices for delivering at least two drug agents from separate reservoirs. Such drug agents may comprise a first and a second medicament. The medical device includes a dose setting mechanism for delivering the drug automatically or manually by the user. An example of prior art devices is given in US 5 378 233. In particular, the present invention relates to a component assembly for a dispense interface of such a medical drug delivery device.

The medical device can be an injector, for example a hand-held injector, especially a pen-type injector, that is an injector of the kind that provides for administration by injection of medicinal products from one or more multidose cartridges. In particular, the present invention relates to such injectors where a user may set the dose.

The drug agents may be contained in two or more multiple dose reservoirs, containers or packages, each containing independent (single drug compound) or pre-mixed (co-formulated multiple drug compounds) drug agents.

Certain disease states require treatment using one or more different medicaments. Some drug compounds need to be delivered in a specific relationship with each other in order to deliver the optimum therapeutic dose. The present patent application is of particular benefit where combination therapy is desirable, but not possible in a single formulation for reasons such as, but not limited to, stability, compromised therapeutic performance and toxicology.

For example, in some cases it may be beneficial to treat a diabetic with a long acting insulin (also may be referred to as the first or primary medicament) along with a glucagon-like peptide-1 such as GLP-1 or GLP-1 analog (also may be referred to as the second drug or secondary medicament).

Accordingly, there exists a need to provide devices for the delivery of two or more medicaments in a single injection or delivery step that is simple for the user to perform without complicated physical manipulations of the drug delivery device. The proposed drug delivery device provides separate storage containers,or cartridge retainers for two or more active drug agents. These active drug agents are then combined and/or delivered to the patient during a single delivery procedure. These active agents may be administered together in a combined dose or alternatively, these active agents may be combined in a sequential manner, one after the other.

The drug delivery device also allows for the opportunity of varying the quantity of the medicaments. For example, one fluid quantity can be varied by changing the properties of the injection device (e.g., setting a user variable dose or changing the device's "fixed" dose). The second medicament quantity can be changed by manufacturing a variety of secondary drug containing packages with each variant containing a different volume and/or concentration of the second active agent.

The drug delivery device may have a single dispense interface. This interface may be configured for fluid communication with a primary reservoir and with a secondary reservoir of medicament containing at least one drug agent. The drug dispense interface can be a type of outlet that allows the two or more medicaments to exit the system and be delivered to the patient.

The combination of compounds from separate reservoirs can be delivered to the body via a double-ended needle assembly. This provides a combination drug injection system that, from a user's perspective, achieves drug delivery in a manner that closely matches the currently available injection devices that use standard needle assemblies. One possible delivery procedure may involve the following steps:
1. Attach a dispense interface to a distal end of the electro-mechanical injection device. The dispense interface comprises a first and a second proximal needle. The first and second needles pierce a first reservoir containing a primary compound and a second reservoir containing a secondary compound, respectively.
2. Attach a dose dispenser, such as a double-ended needle assembly, to a distal end of the dispense interface. In this manner, a proximal end of the needle assembly is in fluidic communication with both the primary compound and secondary compound.
3. Dial up/set a desired dose of the primary compound from the injection device, for example, via a graphical user interface (GUI).
4. After the user sets the dose of the primary compound, the micro-processor controlled control unit may determine or compute a dose of the secondary compound and preferably may determine or compute this second dose based on a previously stored therapeutic dose profile. It is this computed combination of medicaments that will then be injected by the user. The therapeutic dose profile may be user selectable. Alternatively, the user can dial or set a desired dose of the secondary compound.
5. Optionally, after the second dose has been set, the device may be placed in an armed condition. The optional armed condition may be achieved by pressing and/or holding an "OK" or an "Arm" button on a control panel. The armed condition may be provided for a predefined period of time during which the device can be used to dispense the combined dose.
6. Then, the user will insert or apply the distal end of the dose dispenser (e.g., a double ended needle assembly) into the desired injection site. The dose of the combination of the primary compound and the secondary compound (and potentially a third medicament) is administered by activating an injection user interface (e.g., an injection button).

Both medicaments may be delivered via one injection needle or dose dispenser and in one injection step. This offers a convenient benefit to the user in terms of reduced user steps compared to administering two separate injections.

The dispense interface of the drug delivery device represents the structure in which the fluid path of both fluids to be delivered, i.e. the medicaments, is merged before being injected through a single needle. The release of the first and second fluids from the respective cartridges into the dispense interface is controlled by respective valves. The operation of these valves determines the dosage from the fluid associated with that valve's cartridge. The volume downstream from the valves traversed by the fluids before the actual point of injection is also called ullage. The ullage comprises on the one hand the volume of channels specifically dedicated to one of the fluids to be injected and on the other hand also the volume of a channel used by both fluids. In particular, the channel immediately upstream from the entry point to the needle will generally be traversed by all fluids to be injected.

For certain medicaments, the ability to ensure a precise dosage is important. Since the dosage is fundamentally controlled by the amount of fluid released by the valves, the greater the volume of the ullage is, the less difficult it becomes to ensure an exact dosage.

Thus it is an object of the invention to provide a dispense interface for a drug delivery device or a component thereof with which the dosage accuracy of a drug delivery device for delivering medicaments can be improved.

This problem of the invention is solved by an apparatus comprising an inner body of a dispense interface and a manifold , wherein the inner body comprises a recess configured to receive a distal needle from a distal end of the inner body, a first fluid reservoir and a second fluid reservoir, and wherein the inner body and the manifold are assembled such that there is formed a channel providing a fluid connection from the first fluid reservoir and the second fluid reservoir, respectively, to the recess, wherein the manifold comprises a filling block configured to redirect flow, a fluid groove arrangement configured to provide a fluid connection to the filling block, and the fluid connection from the first fluid reservoir and the second fluid reservoir, respectively, to the recess is provided via the fluid groove arrangement and the filling block.

The inner body and the manifold are each respective moulded components. In particular, the inner body and the manifold may be moulded using an open-and-shut-tool. They are then assembled with each other, for example by means of a welding process. Subsequently, the combined inner body and manifold is fitted with other components of the dispense interface. In particular, the outwardly protruding member on the inner body forms an interference fit with an outer body of the dispense interface on assembly.

The function of the assembled inner body and manifold is to provide a fluid channel from each of the multiple cartridges to a common recess which then receives the injection needle. The respective fluids from the cartridges enter the reservoirs of the inner body via individual valves. The manifold provides a covering for each reservoir when the inner body and the manifold are assembled. Through a channel each fluid flows to the recess common to all fluids. By assembling the inner body with the manifold, the inner body serves as covering. Therefore assembling the inner body and the manifold actually creates the fluid channel from each fluid reservoir to the recess. The channel proper may be understood to be the fluid connection from the reservoirs to the recess but not including the recess itself. The recess houses the needle of the drug injection device when it is fully assembled, therefore the fluids enter the needle from the recess.

By forming the fluid channel through an assembly of the two mould-cast components, namely the inner body and the manifold, and being able to mould each component through an open-and-shut tool, a tight mechanical tolerance can be achieved without the need for complex tooling. These tight tolerances consequently allow reducing the dimensions of a number of geometries that determine the ullage volume, including the fluid channel, the recess and the needle wells.

The manifold may be a component to guide fluid flow in the dispense interface of a drug delivery device. The manifold may be any three-dimensional structure. It may in particular be a flat, plate-like structure of arbitrary shape with any indentations, projections or more complicated arrangements on its surface. The manifold may be of any material, in particular a synthetic material.

The fluid channel is formed in particular by the fluid groove arrangement on the manifold. Thereby the inner body serves as covering for the fluid groove arrangement. The manifold provides a filling block, i.e. a structure on its surface, serving to redirect flow from the fluid groove arrangement to the recess. The fluid groove arrangement may comprise any number of fluid grooves, which may be any indentations on the surface of the manifold which permit the passing of fluid along the surface of the manifold.

In a still further preferred embodiment of the invention, the inner body comprises a cavity with a fluid connection to the recess, which cavity is arranged on an axis defined by the recess and wherein the filling block comprises a protrusion, and wherein the inner body and the manifold are assembled such that the cavity receives the filling block. The fluid flowing through the fluid groove arrangement is redirected by the filling block into the cavity, from where it flows further downstream to the recess. When the cavity receives the filling block and in particular the protrusion of the filling block, the effective volume of the cavity is reduced, thereby also reducing the ullage. In terms of production tolerances, it is easier and less costly to reduce the effective volume by having two separate interlocking parts as opposed to trying to produce a volume that is small to begin with in a single moulded component. Thereby a small ullage is achieved by more affordable measures.

In yet a further preferred embodiment of the invention, the protrusion is configured to fill more than half of a volume of the cavity.

In another preferred embodiment of the invention, the inner body comprises at least one outwardly protruding member configured to form an interference fit with an outer body of the dispense interface. The outwardly protruding member is used for a snap fit with the outer body of the dispense interface. The outer body of the dispense interface is a further component of the dispense interface. The outer body is assembled with the inner body and therefore indirectly with the manifold during assembly of the drug delivery device.

By moulding a key assembly snap feature directly on the same component providing the fluid channel and therefore the ullage, tolerance stack-up is prevented. Therefore tighter tolerances can be used compared the case in which the protruding member for the snap fit is provided on a separate component. These tighter tolerances correspond to a reduction in ullage volume.

In another preferred embodiment of the invention, the inner body comprises a flat surface and the recess is arranged on a plane of symmetry of the flat surface, which plane of symmetry is parallel to a longitudinal axis of the inner body. The flat surface of the inner body may face the manifold when the inner body is assembled with the manifold. The plane of symmetry of the flat surface is an imaginary plane and not an actual physical surface.

Because there are at least two fluid valves in the dispense interface, these will likely be away from the central axis of the dispense interface. Having the recess, which represents or is close to the point of entry for both fluids to the needle, on or near the central axis, reduces the total distance in the fluid path and thereby the ullage.

In a yet further preferred embodiment of the invention, the first fluid reservoir and the second fluid reservoir are arranged symmetrically with respect to the plane of symmetry of the flat surface. Since each fluid has to traverse the distance from its corresponding fluid reservoir to the recess, the features of this embodiment help to minimize both the difference in fluid channel length for both fluids and the total fluid channel length for both fluids.

In another preferred embodiment of the invention, the channel is formed at a boundary between the manifold and the inner body. This means that the fluid channel is bounded on one side by the manifold and on the other side by the inner body. This means also that any point in the fluid flow from the fluid groove arrangement up until - but not including - the recess, lies on the boundary between the manifold and the inner body. This arrangement allows for even tighter tolerances and a further reduction of the dimensions of all geometries that determine the ullage volume, including the fluid channel, the recess and the needle wells. In particular, fragile core pins or split lines within the flow path can be avoided.

In yet another preferred embodiment of the invention, the inner body and the manifold are assembled by laser-welding.

In a further preferred embodiment of the invention, the inner body comprises a first aperture and a second aperture each configured to receive a first proximal needle and a second proximal needle, respectively, for providing a respective fluid connection to the first fluid reservoir and the second fluid reservoir. The first proximal needle may be received by the first aperture such that fluid flowing out of the first proximal needle flows into the first fluid reservoir. Likewise, the second proximal needle may be received by the second aperture such that fluid flowing out of the second proximal needle flows into the second fluid reservoir.

In an additional preferred embodiment of the invention, the first aperture and the second aperture are arranged symmetrically with respect to the plane of symmetry of the flat surface. In the same way as for the fluid reservoirs, a symmetric arrangement of the apertures helps to minimize both the difference in fluid channel length for both fluids and the total fluid channel length for both fluids.

In yet a further preferred embodiment of the invention, the apparatus comprises a needle guide arranged at a distal end of the inner body, which needle guide comprises a needle recess. The needle guide is not part of the inner body, but instead comprised in an outer housing. This needle guide is configured to receive the distal needle, i.e. the injection needle. The injection needle passes through the needle guide and is further received by the inner body. The use of the needle guide permits a more precise alignment of the injection needle than would be possible if only the inner body itself were used for alignment.

In another preferred embodiment of the invention, the needle guide is configured to align a distal needle to the recess. This allows using reduced tolerances on the needle position and therefore a smaller channel for the distal needle's entry into the inner body.

In a further preferred embodiment of the invention, the apparatus comprises a pierceable septum arranged along a flat surface of a distal neck portion of the inner body and configured to receive a needle passing the needle guide. The flat surface of the distal neck portion of the inner body may be arranged perpendicular to the longitudinal axis of the inner body.

In another preferred embodiment of the invention, the apparatus comprises a ferrule arranged over the septum and over an outer diameter of the distal neck portion of the inner body. The invention is further directed a drug delivery device comprising an apparatus according to any of the aforementioned embodiments.

These as well as other advantages of various aspects of the present invention will become apparent to those of ordinary skill in the art by reading the following detailed description, with appropriate reference to the accompanying drawings, in which:
Fig. 1 illustrates a perspective view of a delivery device with an end cap of the device removed;
Fig. 2 illustrates a perspective view of the delivery device distal end showing the cartridge;
Fig. 3 illustrates a perspective view of the delivery device illustrated in Fig. 1 or 2 with one cartridge retainer in an open position;
Fig. 4 illustrates a dispense interface and a dose dispenser that may be removably mounted on a distal end of the delivery device illustrated in Fig. 1;
Fig. 5 illustrates the dispense interface and the dose dispenser illustrated in Fig. 4 mounted on a distal end of the delivery device illustrated in Fig. 1;
Fig. 6 illustrates one arrangement of needle assembly that may be mounted on a distal end of the delivery device;
Fig. 7 illustrates a perspective view of the dispense interface illustrated in Fig. 4;
Fig. 8 illustrates another perspective view of the dispense interface illustrated in Fig. 4;
Fig. 9 illustrates a cross-sectional view of the dispense interface illustrated in Fig. 4;
Fig. 10 illustrates an exploded view of the dispense interface illustrated in Fig. 4;
Fig. 11 illustrates a cross-sectional view of the dispense interface and needle assembly mounted onto a drug delivery device, such as the device illustrated in Fig. 1;
Fig. 12 illustrates a perspective view of an alternative dispense interface;
Fig. 13 illustrates another perspective view of the dispense interface illustrated in Fig. 12;
Fig. 14 illustrates a cross sectional view of the dispense interface illustrated in Fig. 12-13;
Fig. 15 illustrates an exploded view of the dispense interface illustrated in Fig. 12-13;
Fig. 16 illustrates an alternative exploded view of the dispense interface illustrated in Fig. 12-13;
Fig. 17 illustrates a perspective view of a main outer body of the dispense interface illustrated in Fig. 12-13;
Fig. 18 illustrates a perspective view of an inner body of the dispense interface illustrated in Fig. 12-13;
Fig. 19 illustrates a perspective view of a manifold of the dispense interface illustrated in Fig. 12-13;
Fig. 20 illustrates a section view of a portion of a distal end of the inner body illustrated in Fig. 19 including a septum, a ferrule and a needle guide;
Fig. 21 illustrates the manifold illustrated in Fig. 19 lased welded to the inner body illustrated in Fig. 18;
Fig. 22 illustrates a perspective view of the needle guide illustrated in Fig. 15 and 16;
Fig. 23 illustrates another perspective view of the needle guide illustrated in Fig. 15 and 16.

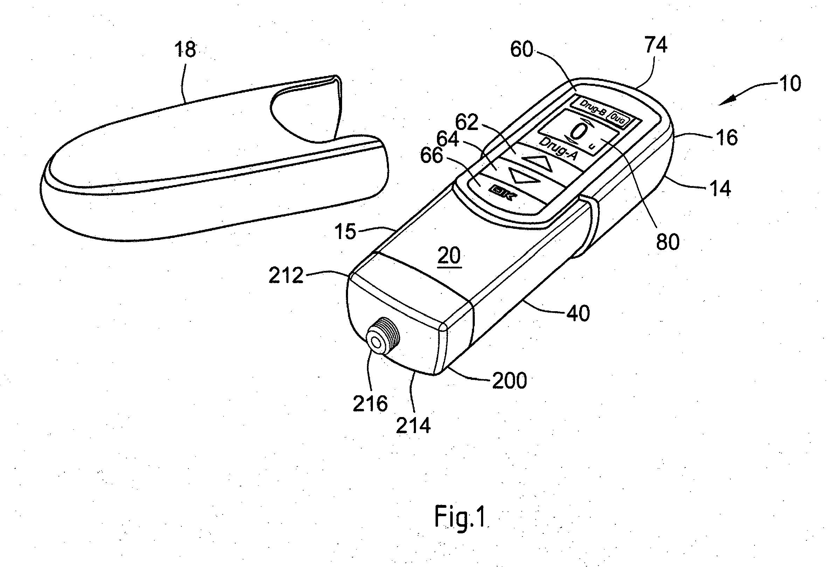

The drug delivery device illustrated in Fig. 1 comprises a main body 14 that extends from a proximal end 16 to a distal end 15. At the distal end 15, a removable end cap or cover 18 is provided. This end cap 18 and the distal end 15 of the main body 14 work together to provide a snap fit or form fit connection so that once the cover 18 is slid onto the distal end 15 of the main body 14, this frictional fit between the cap and the main body outer surface 20 prevents the cover from inadvertently falling off the main body.

The main body 14 contains a micro-processor control unit, an electro-mechanical drive train, and at least two medicament reservoirs. When the end cap or cover 18 is removed from the device 10 (as illustrated in Fig. 1), a dispense interface 200 is mounted to the distal end 15 of the main body 14, and a dose dispenser (e.g., a needle assembly) is attached to the interface. The drug delivery device 10 can be used to administer a computed dose of a second medicament (secondary drug compound) and a variable dose of a first medicament (primary drug compound) through a single needle assembly, such as a double ended needle assembly.

The drive train may exert a pressure on the bung of each cartridge, respectively, in order to expel the doses of the first and second medicaments. For example, a piston rod may push the bung of a cartridge forward a pre-determined amount for a single dose of medicament. When the cartridge is empty, the piston rod is retracted completely inside the main body 14, so that the empty cartridge can be removed and a new cartridge can be inserted.

A control panel region 60 is provided near the proximal end of the main body 14. Preferably, this control panel region 60 comprises a digital display 80 along with a plurality of human interface elements that can be manipulated by a user to set and inject a combined dose. In this arrangement, the control panel region comprises a first dose setting button 62, a second dose setting button 64 and a third button 66 designated with the symbol "OK." In addition, along the most proximal end of the main body, an injection button 74 is also provided (not visible in the perspective view of Fig. 1).

The cartridge holder 40 can be removably attached to the main body 14 and may contain at least two cartridge retainers 50 and 52. Each retainer is configured so as to contain one medicament reservoir, such as a glass cartridge. Preferably, each cartridge contains a different medicament.

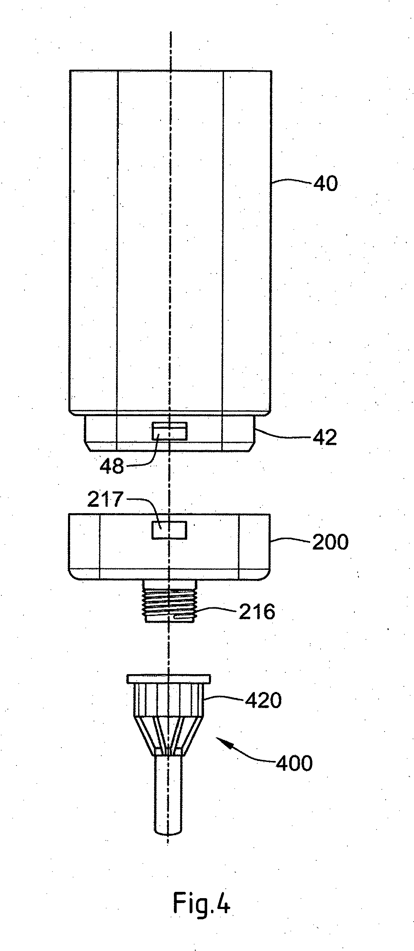

In addition, at the distal end of the cartridge holder 40, the drug delivery device illustrated in Fig. 1 includes a dispense interface 200. As will be described in relation to Fig. 4, in one arrangement, this dispense interface 200 includes a main outer body 212 that is removably attached to a distal end 42 of the cartridge housing 40. As can be seen in Fig. 1, a distal end 214 of the dispense interface 200 preferably comprises a needle hub 216. This needle hub 216 may be configured so as to allow a dose dispenser, such as a conventional pen type injection needle assembly, to be removably mounted to the drug delivery device 10.

Once the device is turned on, the digital display 80 shown in Fig. 1 illuminates and provides the user certain device information, preferably information relating to the medicaments contained within the cartridge holder 40. For example, the user is provided with certain information relating to both the primary medicament (Drug A) and the secondary medicament (Drug B).

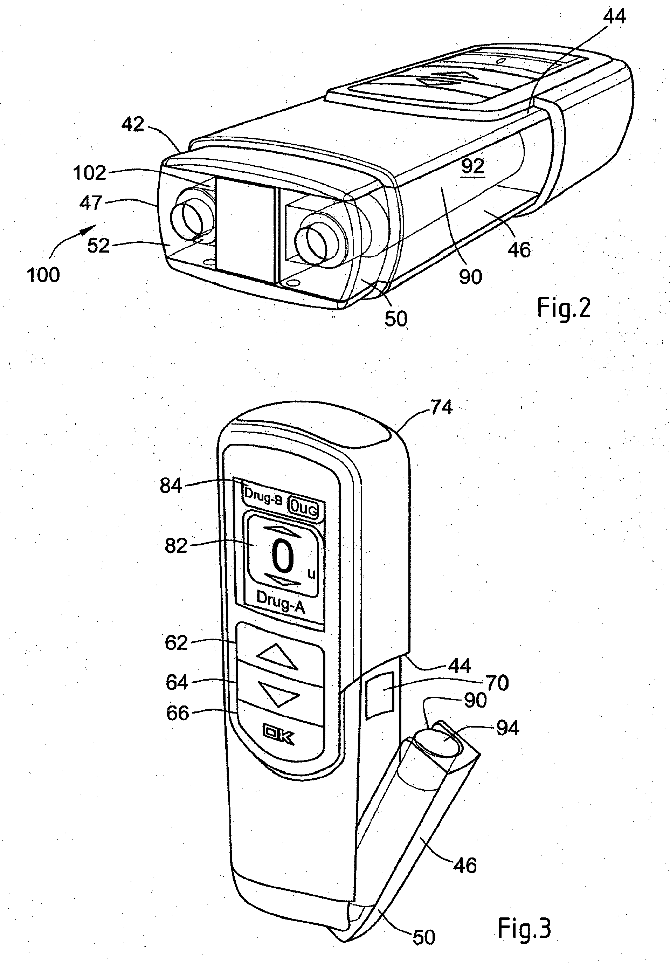

As shown in Fig. 3, the first and second cartridge retainers 50, 52 maybe hinged cartridge retainers. These hinged retainers allow user access to the cartridges. Fig. 3 illustrates a perspective view of the cartridge holder 40 illustrated in Fig. 1 with the first hinged cartridge retainer 50 in an open position. Fig. 3 illustrates how a user might access the first cartridge 90 by opening up the first retainer 50 and thereby having access to the first cartridge 90.

As mentioned above when discussing Fig. 1, a dispense interface 200 is coupled to the distal end of the cartridge holder 40. Fig. 4 illustrates a flat view of the dispense interface 200 unconnected to the distal end of the cartridge holder 40. A dose dispenser or needle assembly that may be used with the interface 200 is also illustrated and is provided in a protective outer cap 420.

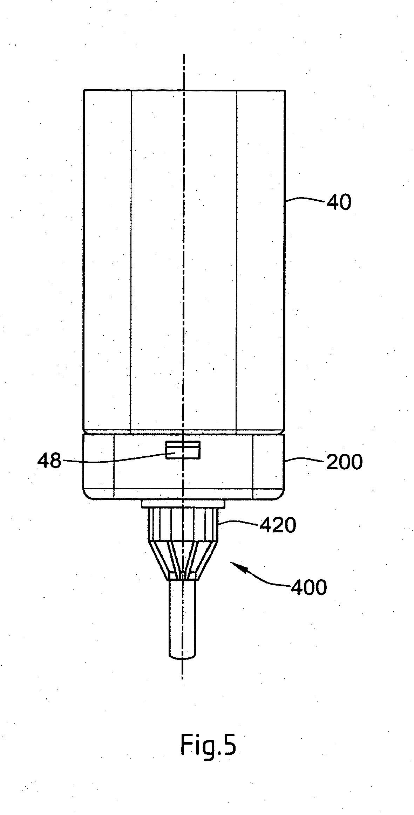

In Fig. 5, the dispense interface 200 illustrated in Fig. 4 is shown coupled to the cartridge holder 40. The axial attachment means between the dispense interface 200 and the cartridge holder 40 can be any known axial attachment means to those skilled in the art, including snap locks, snap fits, snap rings, keyed slots, and combinations of such connections. The connection or attachment between the dispense interface and the cartridge holder may also contain additional features (not shown), such as connectors, stops, splines, ribs, grooves, pips, clips and the like design features, that ensure that specific hubs are attachable only to matching drug delivery devices. Such additional features would prevent the insertion of a non-appropriate secondary cartridge to a non-matching injection device.

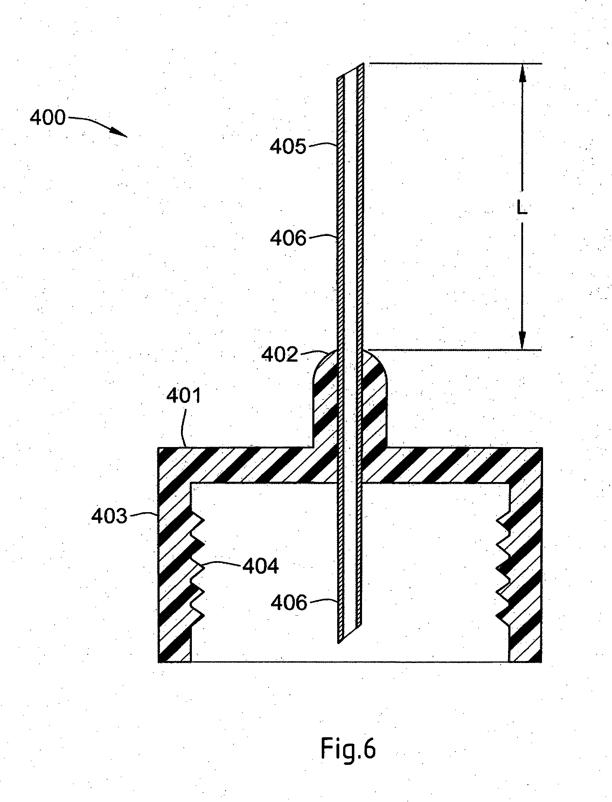

Fig. 5 also illustrates the needle assembly 400 and protective cover 420 coupled to the distal end of the dispense interface 200 that may be screwed onto the needle hub of the interface 200. Fig. 6 illustrates a cross sectional view of the double ended needle assembly 402 mounted on the dispense interface 200 in Fig. 5.

The needle assembly 400 illustrated in Fig. 6 comprises a double ended needle 406 and a hub 401. The double ended needle or cannula 406 is fixedly mounted in a needle hub 401. This needle hub 401 comprises a circular disk shaped element which has along its periphery a circumferential depending sleeve 403. Along an inner wall of this hub member 401, a thread 404 is provided. This thread 404 allows the needle hub 401 to be screwed onto the dispense interface 200 which, in one preferred arrangement, is provided with a corresponding outer thread along a distal hub. At a center portion of the hub element 401 there is provided a protrusion 402. This protrusion 402 projects from the hub in an opposite direction of the sleeve member. A double ended needle 406 is mounted centrally through the protrusion 402 and the needle hub 401. This double ended needle 406 is mounted such that a first or distal piercing end 405 of the double ended needle forms an injecting part for piercing an injection site (e.g., the skin of a user).

Similarly, a second or proximal piercing end 406 of the needle assembly 400 protrudes from an opposite side of the circular disc so that it is concentrically surrounded by the sleeve 403. In one needle assembly arrangement, the second or proximal piercing end 406 may be shorter than the sleeve 403 so that this sleeve to some extent protects the pointed end of the back sleeve. The needle cover cap 420 illustrated in Fig. 4 and 5 provides a form fit around the outer surface 403 of the hub 401.
Referring now to Fig. 4 to 11, one preferred arrangement of this interface 200 will now be discussed. In this one preferred arrangement, this interface 200 comprises:
a. a main outer body 210,
b. an first inner body 220,
c. a second inner body 230,
d. a first piercing needle 240,
e. a second piercing needle 250,
f. a valve seal 260, and
g. a septum 270.

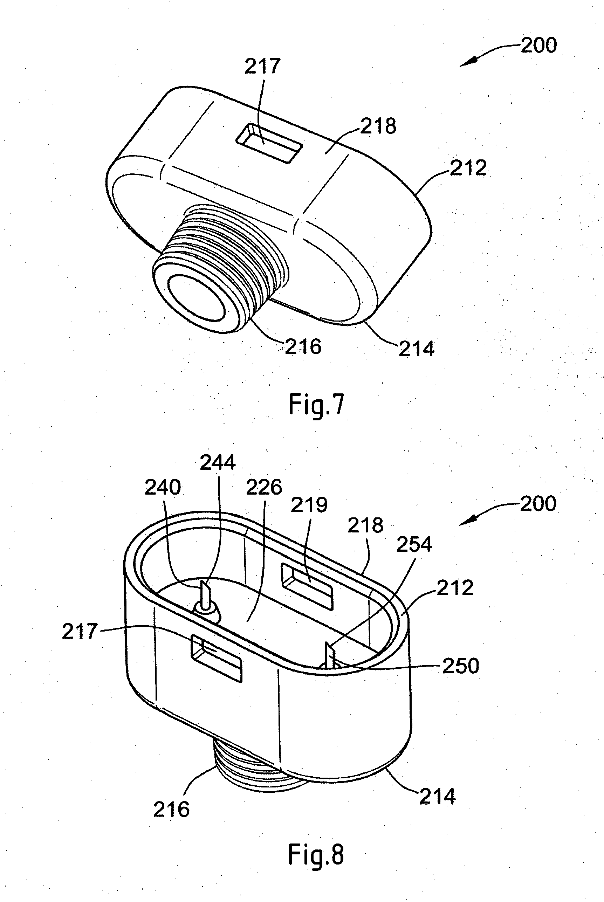

The main outer body 210 comprises a main body proximal end 212 and a main body distal end 214. At the proximal end 212 of the outer body 210, a connecting member is configured so as to allow the dispense interface 200 to be attached to the distal end of the cartridge holder 40. Preferably, the connecting member is configured so as to allow the dispense interface 200 to be removably connected the cartridge holder 40. In one preferred interface arrangement, the proximal end of the interface 200 is configured with an upwardly extending wall 218 having at least one recess. For example, as may be seen from Fig. 8, the upwardly extending wall 218 comprises at least a first recess 217 and a second recess 219.

Preferably, the first and the second recesses 217, 219 are positioned within this main outer body wall so as to cooperate with an outwardly protruding member located near the distal end of the cartridge housing 40 of the drug delivery device 10. For example, this outwardly protruding member 48 of the cartridge housing may be seen in Fig. 4 and 5. A second similar protruding member is provided on the opposite side of the cartridge housing. As such, when the interface 200 is axially slid over the distal end of the cartridge housing 40, the outwardly protruding members will cooperate with the first and second recess 217, 219 to form an interference fit, form fit, or snap lock. Alternatively, and as those of skill in the art will recognize, any other similar connection mechanism that allows for the dispense interface and the cartridge housing 40 to be axially coupled could be used as well.

The main outer body 210 and the distal end of the cartridge holder 40 act to form an axially engaging snap lock or snap fit arrangement that could be axially slid onto the distal end of the cartridge housing. In one alternative arrangement, the dispense interface 200 may be provided with a coding feature so as to prevent inadvertent dispense interface cross use. That is, the inner body of the hub could be geometrically configured so as to prevent an inadvertent cross use of one or more dispense interfaces. A mounting hub is provided at a distal end of the main outer body 210 of the dispense interface 200. Such a mounting hub can be configured to be releasably connected to a needle assembly. As just one example, this connecting means 216 may comprise an outer thread that engages an inner thread provided along an inner wall surface of a needle hub of a needle assembly, such as the needle assembly 400 illustrated in Fig. 6. Alternative releasable connectors may also be provided such as a snap lock, a snap lock released through threads, a bayonet lock, a form fit, or other similar connection arrangements.

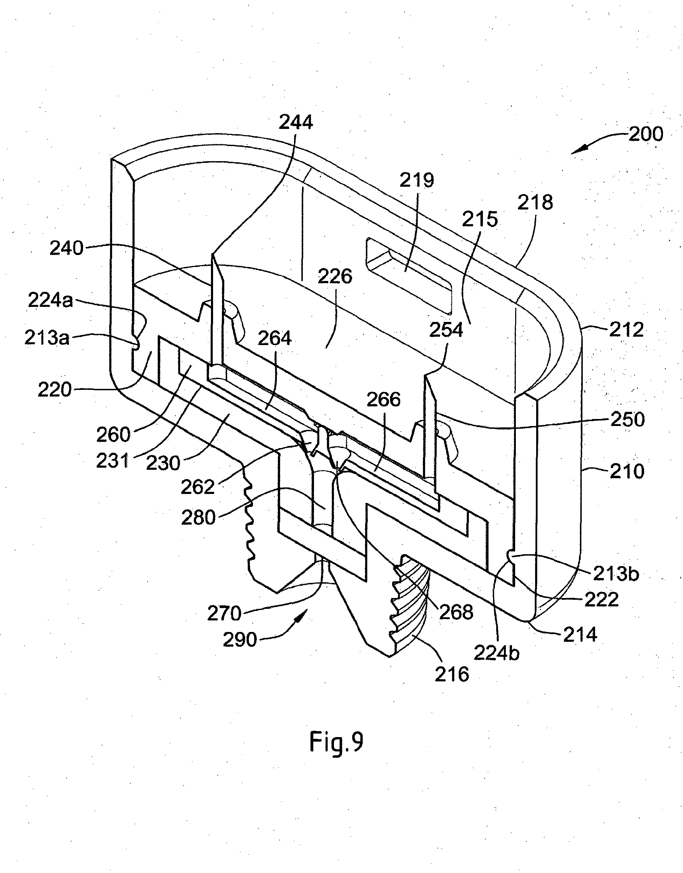

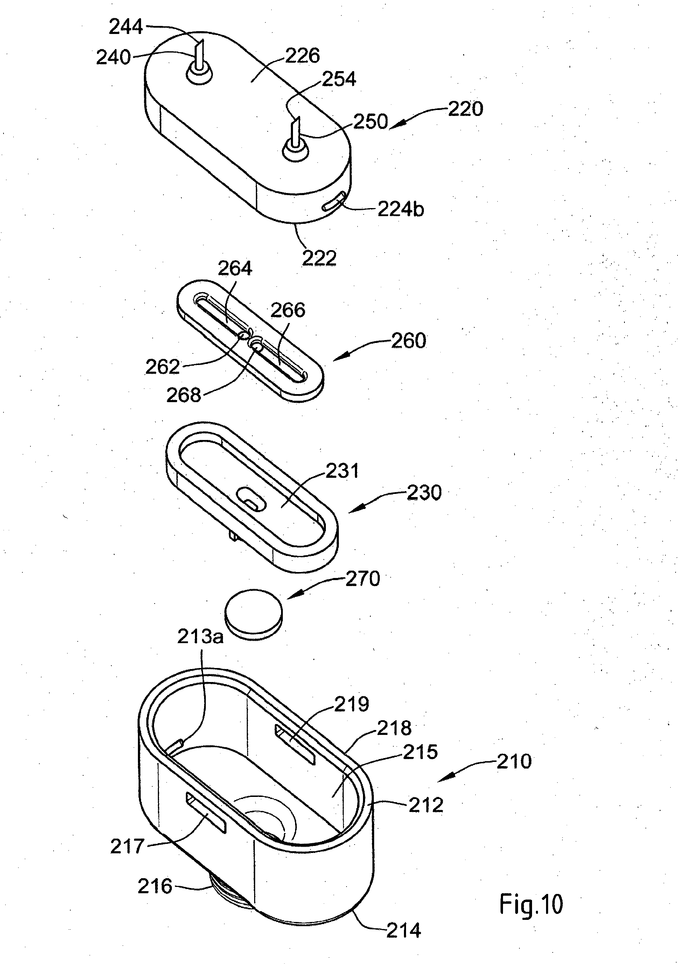

The dispense interface 200 further comprises a first inner body 220. Certain details of this inner body are illustrated in Fig. 8-11. Preferably, this first inner body 220 is coupled to an inner surface 215 of the extending wall 218 of the main outer body 210. More preferably, this first inner body 220 is coupled by way of a rib and groove form fit arrangement to an inner surface of the outer body 210. For example, as can be seen from Fig. 9, the extending wall 218 of the main outer body 210 is provided with a first rib 213a and a second rib 213b. This first rib 213a is also illustrated in Fig. 10. These ribs 213a and 213b are positioned along the inner surface 215 of the wall 218 of the outer body 210 and create a form fit or snap lock engagement with cooperating grooves 224a and 224b of the first inner body 220. In a preferred arrangement, these cooperating grooves 224a and 224b are provided along an outer surface 222 of the first inner body 220.

In addition, as can be seen in Fig. 8-10, a proximal surface 226 near the proximal end of the first inner body 220 may be configured with at least a first proximally positioned piercing needle 240 comprising a proximal piercing end portion 244. Similarly, the first inner body 220 is configured with a second proximally positioned piercing needle 250 comprising a proximally piercing end portion 254. Both the first and second needles 240, 250 are rigidly mounted on the proximal surface 226 of the first inner body 220.

Preferably, this dispense interface 200 further comprises a valve arrangement. Such a valve arrangement could be constructed so as to prevent cross contamination of the first and second medicaments contained in the first and second reservoirs, respectively. A preferred valve arrangement may also be configured so as to prevent back flow and cross contamination of the first and second medicaments.

In one preferred system, dispense interface 200 includes a valve arrangement in the form of a valve seal 260. Such a valve seal 260 may be provided within a cavity 231 defined by the second inner body 230, so as to form a holding chamber 280. Preferably, cavity 231 resides along an upper surface of the second inner body 230. This valve seal comprises an upper surface that defines both a first fluid groove 264 and second fluid groove 266. For example, Fig. 9 illustrates the position of the valve seal 260, seated between the first inner body 220 and the second inner body 230. During an injection step, this seal valve 260 helps to prevent the primary medicament in the first pathway from migrating to the secondary medicament in the second pathway, while also preventing the secondary medicament in the second pathway from migrating to the primary medicament in the first pathway. Preferably, this seal valve 260 comprises a first non-return valve 262 and a second non-return valve 268. As such, the first non-return valve 262 prevents fluid transferring along the first fluid pathway 264, for example a groove in the seal valve 260, from returning back into this pathway 264. Similarly, the second non-return valve 268 prevents fluid transferring along the second fluid pathway 266 from returning back into this pathway 266.

Together, the first and second grooves 264, 266 converge towards the non-return valves 262 and 268 respectively, to then provide for an output fluid path or a holding chamber 280. This holding chamber 280 is defined by an inner chamber defined by a distal end of the second inner body both the first and the second non return valves 262, 268 along with a pierceable septum 270. As illustrated, this pierceable septum 270 is positioned between a distal end portion of the second inner body 230 and an inner surface defined by the needle hub of the main outer body 210.

The holding chamber 280 terminates at an outlet port of the interface 200. This outlet port 290 is preferably centrally located in the needle hub of the interface 200 and assists in maintaining the pierceable seal 270 in a stationary position. As such, when a double ended needle assembly is attached to the needle hub of the interface (such as the double ended needle illustrated in Fig. 6), the output fluid path allows both medicaments to be in fluid communication with the attached needle assembly.

The hub interface 200 further comprises a second inner body 230. As can be seen from Fig. 9, this second inner body 230 has an upper surface that defines a recess, and the valve seal 260 is positioned within this recess. Therefore, when the interface 200 is assembled as shown in Fig. 9, the second inner body 230 will be positioned between a distal end of the outer body 210 and the first inner body 220. Together, second inner body 230 and the main outer body hold the septum 270 in place. The distal end of the inner body 230 may also form a cavity or holding chamber that can be configured to be fluid communication with both the first groove 264 and the second groove 266 of the valve seal.

Axially sliding the main outer body 210 over the distal end of the drug delivery device attaches the dispense interface 200 to the multi-use device: In this manner, a fluid communication may be created between the first needle 240 and the second needle 250 with the primary medicament of the first cartridge and the secondary medicament of the second cartridge, respectively.

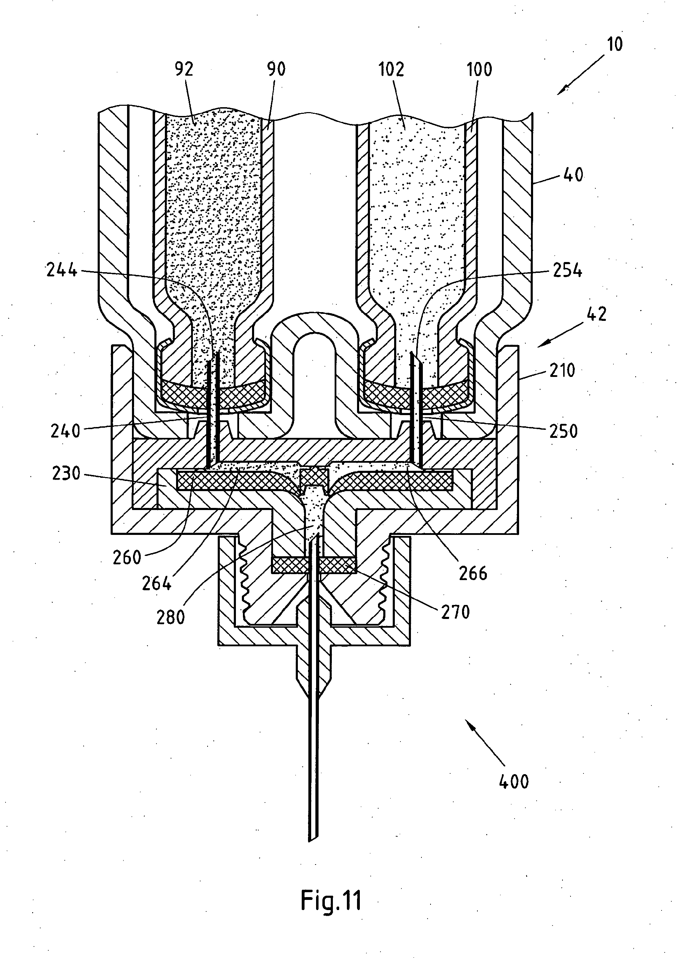

Fig. 11 illustrates the dispense interface 200 after it has been mounted onto the distal end 42 of the cartridge holder 40 of the drug delivery device 10 illustrated in Fig. 1. A double ended needle 400 is also mounted to the distal end of this interface. The cartridge holder 40 is illustrated as having a first cartridge containing a first medicament and a second cartridge containing a second medicament.

When the interface 200 is first mounted over the distal end of the cartridge holder 40, the proximal piercing end 244 of the first piercing needle 240 pierces the septum of the first cartridge 90 and thereby resides in fluid communication with the primary medicament 92 of the first cartridge 90. A distal end of the first piercing needle 240 will also be in fluid communication with a first fluid path groove 264 defined by the valve seal 260.

Similarly, the proximal piercing end 254 of the second piercing needle 250 pierces the septum of the second cartridge 100 and thereby resides in fluid communication with the secondary medicament 102 of the second cartridge 100. A distal end of this second piercing needle 250 will also be in fluid communication with a second fluid path groove 266 defined by the valve seal 260.

Fig. 11 illustrates a preferred arrangement of such a dispense interface 200 that is coupled to a distal end 15 of the main body 14 of drug delivery device 10. Preferably, such a dispense interface 200 is removably coupled to the cartridge holder 40 of the drug delivery device 10.

As illustrated in Fig. 11, the dispense interface 200 is coupled to the distal end of a cartridge housing 40. This cartridge holder 40 is illustrated as containing the first cartridge 90 containing the primary medicament 92 and the second cartridge 100 containing the secondary medicament 102. Once coupled to the cartridge housing 40, the dispense interface 200 essentially provides a mechanism for providing a fluid communication path from the first and second cartridges 90, 100 to the common holding chamber 280. This holding chamber 280 is illustrated as being in fluid communication with a dose dispenser. Here, as illustrated, this dose dispenser comprises the double ended needle assembly 400. As illustrated, the proximal end of the double ended needle assembly is in fluid communication with the chamber 280.

In one preferred arrangement, the dispense interface is configured so that it attaches to the main body in only one orientation, that is it is fitted only one way round. As such as illustrated in Fig. 11, once the dispense interface 200 is attached to the cartridge holder 40, the primary needle 240 can only be used for fluid communication with the primary medicament 92 of the first cartridge 90 and the interface 200 would be prevented from being reattached to the holder 40 so that the primary needle 240 could now be used for fluid communication with the secondary medicament 102 of the second cartridge 100. Such a one way around connecting mechanism may help to reduce potential cross contamination between the two medicaments 92 and 102.

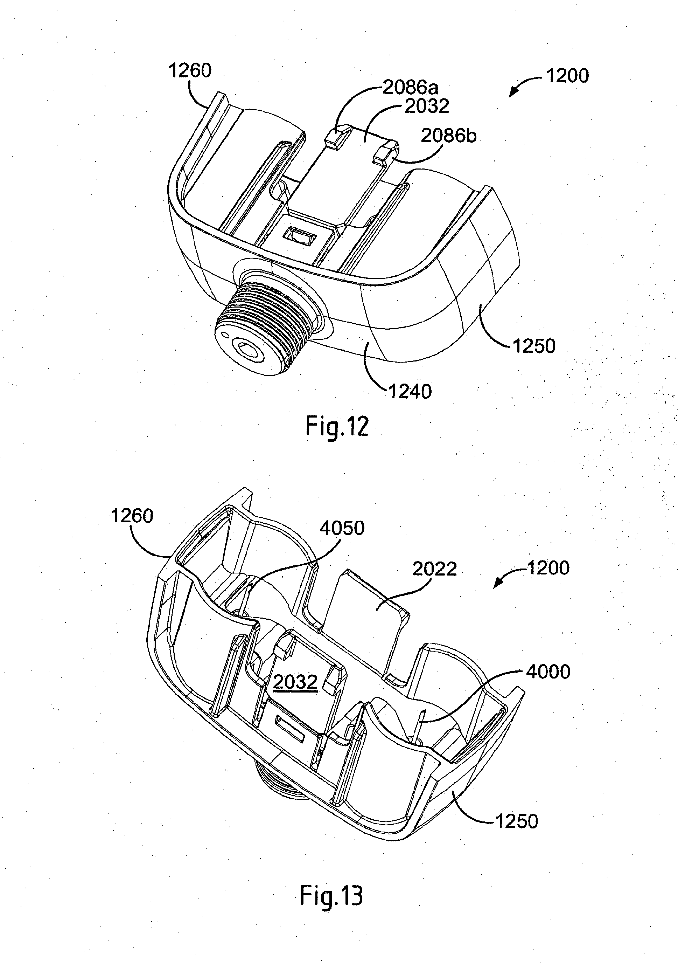

In the following embodiments of the present invention will be described in detail with reference to Fig. 12 to 23.

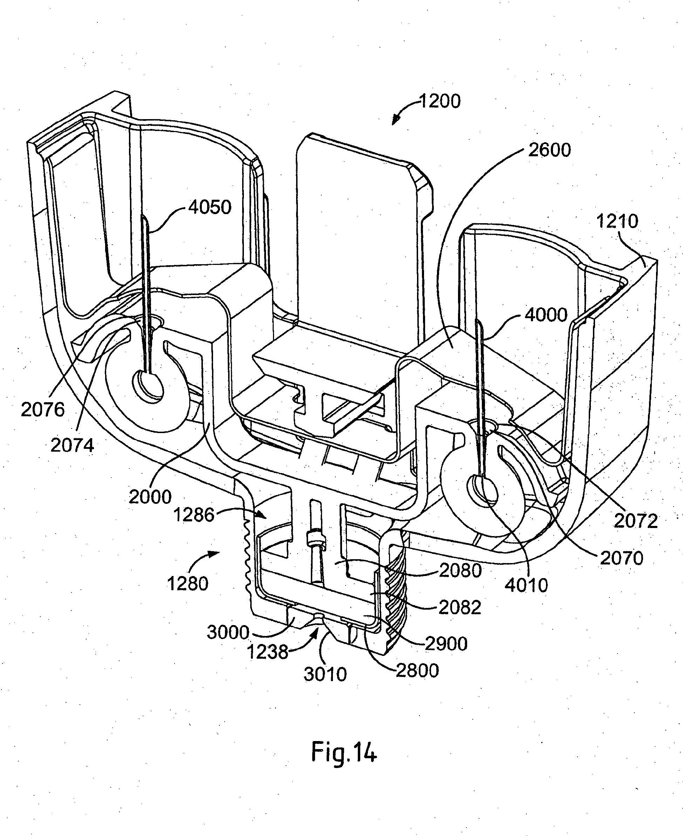

Figure 12 illustrates a perspective distal view of one example embodiment of the dispense interface 1200. Figure 13 illustrates a perspective proximal view of the example embodiment of the dispense interface 1200 illustrated in Figure 12 and Figure 14 illustrates a cross-sectional view of the dispense interface 1200 illustrated in Figures 12 and 13. As will now be discussed in greater detail, in one preferred arrangement, the dispense interface 1200 illustrated in Figures 12-17 comprises:
a. a main outer body 1210;
b. an inner body 2000;
c. a manifold 2300;
d. a first piercing needle 4000;
e. a second piercing needle 4050;
f. a lock-out spring 2600;
g. a first diaphragm valve 2700;
h. a second diaphragm valve 2750;
i. a ferrule 2800;
j. an outer septum 2900; and
k. a needle guide 3000.

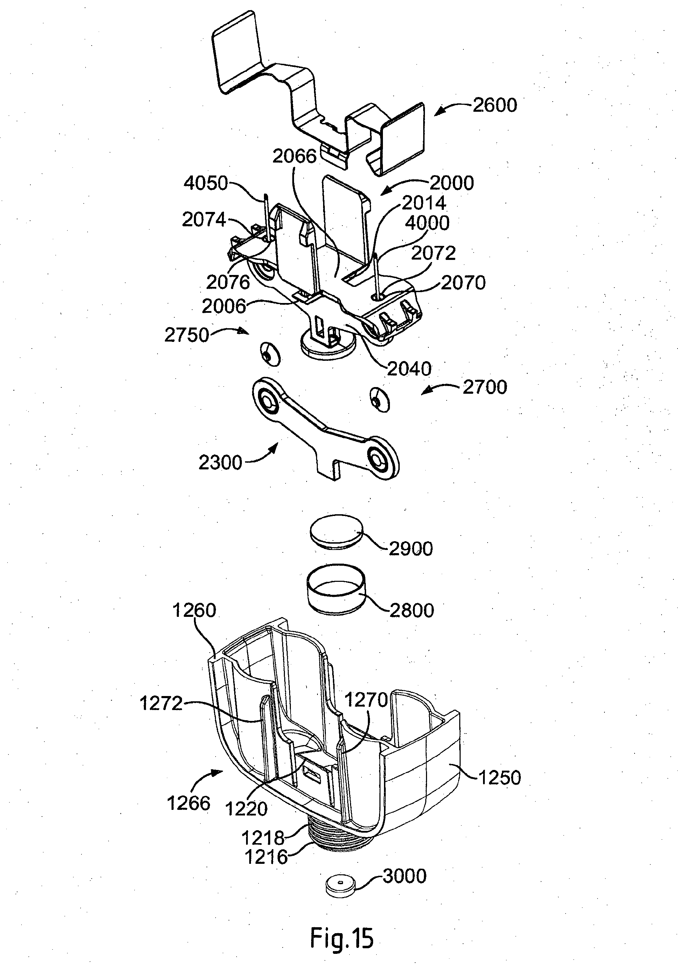

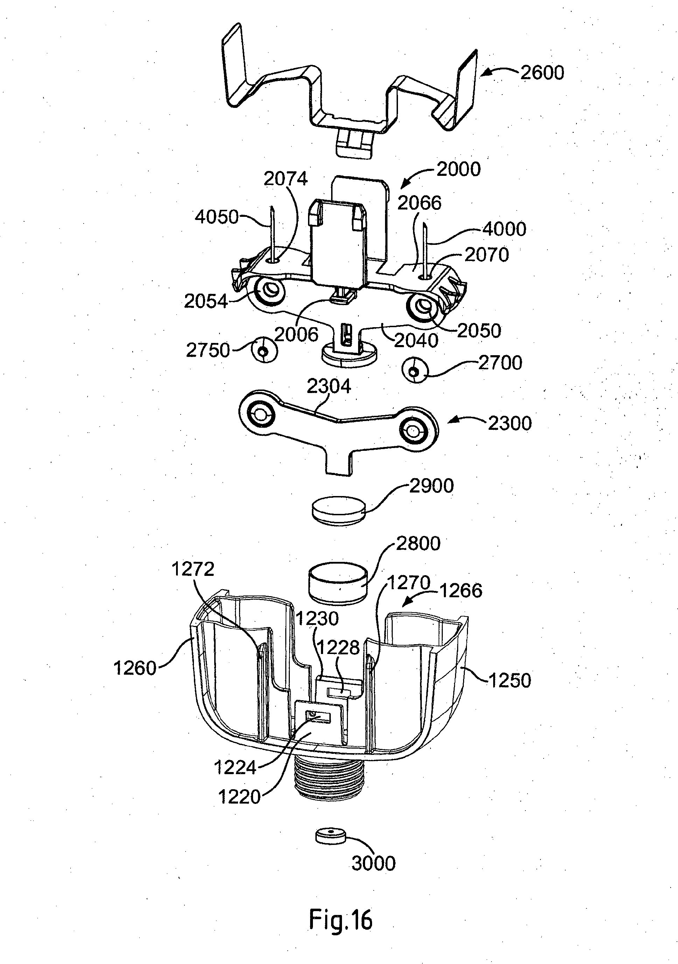

A general interrelationship between these various component parts may be seen from Figure 15 which illustrates one exploded perspective view of the dispense interface 1200. Similarly, Figure 16 illustrates an alternative exploded perspective view of the dispense interface 1200.

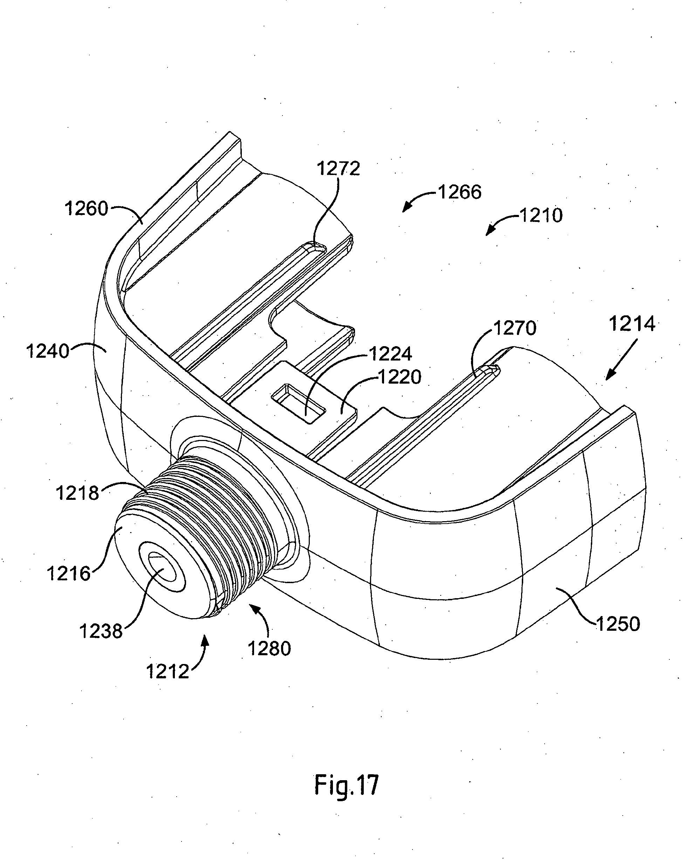

Figure 17 illustrates a perspective view of the main outer body 1210 of the dispense interface 1200. Referring now to Figures 12-17, as illustrated, this body 1210 comprises a main body distal end 1212 and a main body proximal end 1214. The main body proximal end 1214 is configured to be seated along the distal end of the drug delivery device near the distal end of the cartridge holder. Preferably, the main outer body 1210 comprises an injection molded polypropylene (PP) component.

Furthermore, the main body 1210 comprises a first and a second shroud 1250, 1260 extending from the distal end to the proximal end of the main body 1210. Preferably, when the main body is assembled together with the other components of the dispense interface 1200 and the interface is attached to the drug delivery device, shrouds 1250, 1260 obscure the exposed first and second piercing needles or cannulas 4000, 4050 (see, also, e.g., Figure 13). As such, shrouds 1250, 1260 help to prevent needle stick injuries as a user attaches the dispense interface 1200 to the drug delivery device.

As may be seen from Figures 12-17, a top surface 1240 of the outer body 1210 comprises a smooth, rounded outer surface. In this illustrated outer body arrangement, the distal end of the main body of the drug delivery device comprises a two flat portions that, when the dispense interface 1200 is properly connected to the drug delivery device, these two distally facing flat portions cover the front and back areas of the main body 1210 of the dispense interface so that an overall smooth surface will be provided.

In addition and now referring to Figures 12 - 17, the main outer body 1210 further comprises two flexible connecting members 1220, 1230, one on each side of the outer body 1210. For example, the first connecting member 1220 may be seen in Figures 15 to 17 and the second connecting member 1230 may be seen in Figure 16. These connecting members are positioned between the first and second shrouds 1250, 1260. Preferably, these connecting members 1220, 1230 are configured as flat tabs and constructed so as to flex outwardly (i.e., away from one another) so as allow the main outer body 1210 to be attached to and disconnected from an inner body 2000 (see, e.g., Figure 14) of the dispense interface 1200. In one example embodiment, the two connecting members 1220, 1230 extend in a proximal direction with each flat portion comprising at least one recess. For example, as may be seen from Figure 16 or 17, the first extending flat portion 1220 comprises at least a first recess 1224. Similarly, as may be seen from Figure 16, the second extending flat portion 1230 comprises a second recess 1228.

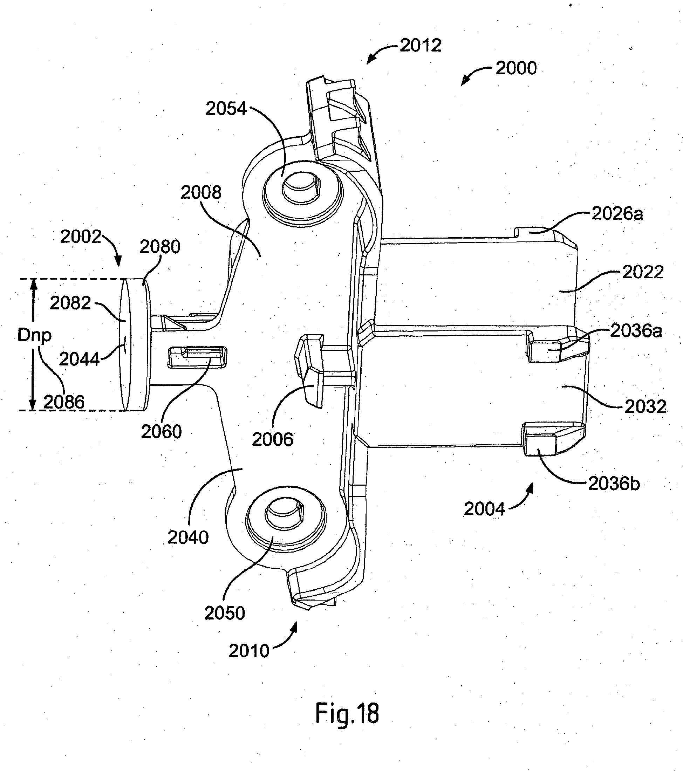

Preferably, the two recesses 1224, 1228 are positioned within this main outer body 1210 so as to cooperate with a first and a second outwardly protruding member 2006, 2014 respectively, located near a middle portion of the inner body 2000. For example, as may be seen from Figure 18 which illustrates a perspective view of an inner body 2000, the inner body 2000 comprises a first outwardly protruding member 2006. A second similar outwardly protruding member 2014 is provided on the opposite side of the inner body portion. These outwardly protruding members 2006, 2014 of the inner body may be seen in Figure 15.

As such, when the main body 1210 is axially positioned over the distal end of the inner body 2000 during an assembly step, the outwardly protruding members 2006, 2014 cooperate with the first and the second recess 1224, 1228 of the main outer body so as to form an interference fit, form fit, or snap lock between the two components. Preferably, such an interference fit comprises a permanent interference fit. Alternatively, and as those of skill in the art will recognize, other similar connection mechanisms that allow for the main outer body 1210 and the inner body 2000 to be axially coupled could be used as well. However, in one preferred arrangement, this connection comprises a permanent interference fit so as to prevent user manipulation of the interface in an attempt to reuse of the dispense interface.

The inner body 2000 and the release button provided at the distal end of the cartridge holder of the device act to form an axially engaging snap lock or snap fit arrangement that could be axially slid onto the distal end of the cartridge housing. In an example embodiment, the dispense interface 1200 may be provided with a coding feature so as to prevent inadvertent dispense interface cross use. That is, the inner body of the hub could be geometrically configured so as to prevent an inadvertent cross use of one or more dispense interfaces.

The outer main body 1210 further comprises a guide arrangement 1266 preferably in the form of a plurality of guide ribs. The guide arrangement improves ease of fitment of the dispense interface 1200 onto the drug delivery device by properly orientating the interface 1200 during attachment. For example, as illustrated in Figures 15-17, two guide ribs 1270, 1272 are shown and they are provided along one side of the main body. The first guide rib 1270 is positioned between the first flat tab 1220 and the first shroud 1250. Similarly, the second guide rib 1272 is also positioned on the same side of the main body as the first rib 1270 and positioned between the first flat tab 1220 and the second shroud 1260. A similar dual guide rib arrangement is provided on the other side of the main body 1210, as shown in Fig. 15.

In this configuration, the guide rib arrangement improves ease of fitment. In one preferred arrangement, the guide rib arrangement 1266 may comprise a symmetric guide rib arrangement, so that the dispense interface may be fitted onto the distal end of the device in either orientation. In an alternative guide rib arrangement 1266, the arrangement comprises a non-symmetric arrangement where the dispense interface would not fit in either orientation to the drug delivery device but only in one single orientation.

Referring back to the main outer body 1200 illustrated in Figure 17, a mounting hub 1216 is provided at the distal end 1212 of the main outer body 1210. Such a mounting hub 1216 may comprise a connecting mechanism 1218. Preferably, this connecting mechanism 1218 allows a needle assembly (such as the double ended needle assembly 400 illustrated in Figure 6) to be releasably connected to the hub 1216. As just one example, this connecting mechanism 1218 may comprise an outer thread that engages an inner thread provided along an inner wall surface of a needle hub of a needle assembly, such as the needle assembly 400 illustrated in Figure 6. Alternative releasable connectors may also be provided such as a snap lock, a snap lock released through threads, a bayonet lock, a form fit, or other similar connection arrangements.

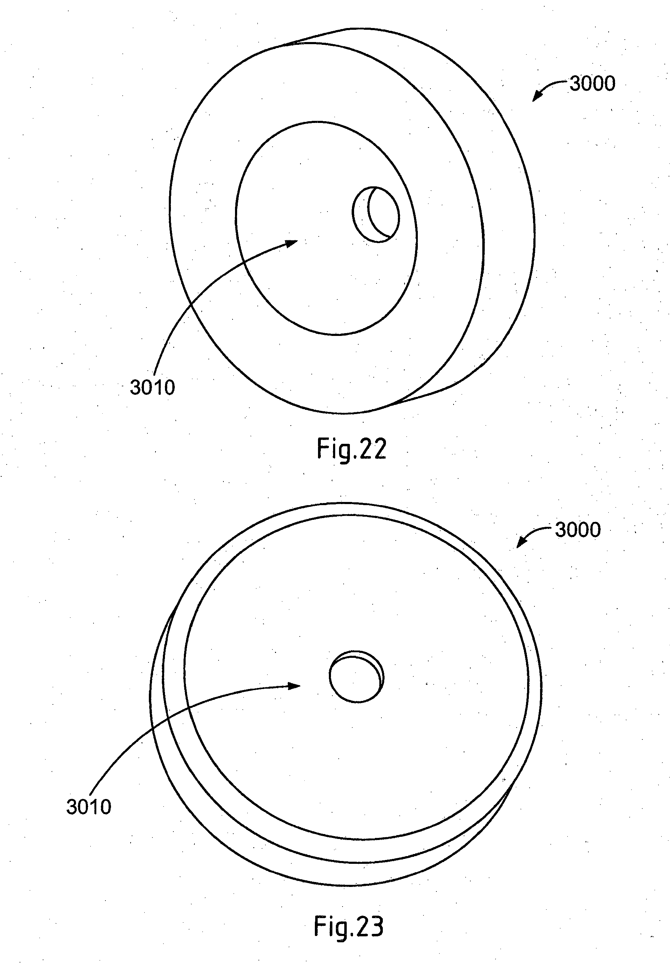

The main body mounting hub 1216 extends distally away from the outer surface 1240 of the outer body and may be generally shaped as a cylindrical extension 1280. This cylindrical extension 1280 defines an interior space 1286. This interior space 1286 may be seen from Figure 14 which provides a cross sectional view of an assembled dispense interface 1200. At its most distal end, the connecting hub defines an aperture 1238. In an example embodiment, this aperture 1238 is appropriately sized for receiving a needle guide 3000. For example, Figures 22 and 23 illustrate perspective views of such a needle guide 3000. Preferably, the needle guide 3000 comprises a generally circular outer shape and this generally circular outer shape defines an inner recess 3010. Preferably, this inner recess 3010 comprises a conical shaped inner recess. One advantage of providing such a conical shaped inner recess is that, when a double ended needle is attached to the mounting hub 1216, the recess will guide the proximal needle of the double ended needle by the conical shaped recess 3010 into contact with a septum provided by the dispense interface 1200. This allows the proximally directed needle of the attached needle assembly to be guided through a contact path in the distal end of the inner body and then eventually into fluid communication with a holding chamber or cavity defined by the inner body 2000. By guiding the proximally directed needle of the needle assembly into a centered position, attachment of the needle assembly can be made easier. Further, the proximally directed needle may be guided into the holding chamber or cavity of the inner body 2000 more precisely. Thus, the diameter and consequently the volume of the holding chamber or cavity can be made smaller than without the conical recess 3010 of the needle guide 3000.

In addition, and as will be explained in greater detail below, the interior space 1286 defined by the cylindrical extension 1280 is appropriately dimensioned so as to securely position and align a ferrule 2800 and an outlet septum 2900 that are seated on a flat distal surface 2082 of a neck portion 2080 provided near a distal end 2002 of the inner body 2000. This is illustrated in the cross sectional view of the dispense interface 1200 provided in Figure 14.

As may also be seen from the exploded views of Figures 15 and 16, aside from the outer body 1200, the dispense interface 1200 further comprises an inner body 2000. For example, Figure 18 illustrates a perspective view of the inner body 2000 illustrated in Figures 15 and 16. As can be seen from Figure 18 and the cross sectional view of the dispense interface 1200 provided in Figure 14, the inner body 2000 is seated within an interior space defined by the outer main body 1210. Preferably, it is the inner body 2000 of the dispense interface 1200 that is configured to be coupled to the distal end of the drug delivery device while also being securely positioned within an interior space defined by the outer body 1210. To accomplish a removable connection to the distal end of the drug delivery device, the inner body 2000 comprises a generally a Y-shaped configuration 2008 and this y-shaped configuration has a distal extending portion 2002 and a proximal extending portion 2004.

In addition, two wing shaped members 2010, 2012 reside between the distal extending portion 2002 and the proximal extending portion 2004. The proximal portion 2004 of the inner body 2000 comprises a first proximally extending tab and a second proximally extending tab 2022, 2032, respectively. These proximally extending tabs 2022, 2032 are made of a generally flexible material so as to allow the tabs to be flexed inwardly towards an inner space (i.e., towards one another) when the dispense interface 1200 is either attached to or removed from the drug delivery device.

The first and second tabs 2022, 2032 further comprise a protrusion arrangement so as to enable the dispense interface 1200 to be releasably coupled to the drug delivery device. For example, the first tab 2022 comprises a first and a second protrusion 2026a, 2026b while the second tab 2032 comprises a first and a second protrusion 2036a, 2036b. When the dispense interface 1200 and hence the inner body 2000 is attached to the drug delivery device, the various protrusions of the tabs 2026a, 2026b and 2036a, 2036b slide under and then into a first and a second corresponding recess provided along the flat surfaces of the main body near the distal end of the outer housing of the drug delivery device. One advantage of such a flexible tab arrangement is that these tabs may be releasably coupled to the main outer body 1210, for example by pressing a release button, so as to help ensure that removing a dispense interface 1200 coupled to a delivery device will not inadvertently disassembly the dispense interface 1200.

With such a tab configuration, a soft ejection may be achieved. For example, pressing the release button on the drug delivery device releases the dispense interface 1200 to a detent position on the drug delivery device. This provides for a two-part controlled ejection of the dispense interface which may still have a double ended needle assembly attached to it. Pressing the release button may also put a detaching force on the dispense interface 1200, so that the dispense interface 1200 may be displaced far enough so as to disengage the first and second piercing needles 4000, 4050 from fluid communication with the first and second cartridges and yet engage the internal lock-out features, such as lock-out spring 2600, to thereby prevent the dispense interface 1200 from being reattached to the drug delivery device. The detent on the drug delivery device holds the dispense interface in this disengaged position and ready for the user to remove it completely from the device and then dispose of it.

In addition, as can be seen from Figures 15-16 and 18, the inner body 2000 further comprises a flat surface 2040 residing between the two wing shaped members 2010, 2012 and residing between the distal portion 2002 and the proximal portion 2004. This generally flat surface 2040 preferably defines a plurality of recesses or reservoirs. For example, in a preferred arrangement, the generally flat surface 2040 may be configured to define both a first circular cavity or first fluid reservoir 2050 and a second circular cavity or second fluid reservoir 2054. In one preferred arrangement, the first and second reservoirs 2050, 2054 may be used in conjunction with a valve arrangement to allow a fluid, such as a medicament, to flow from the first and second piercing needles 4000, 4050 to a holding chamber 2060.

For example, in one preferred arrangement, the first circular cavity or reservoir 2050 will define a certain cavity depth and this first circular cavity may have two different diameters. Similarly, the second circular recess 2054 may also define a certain cavity depth having two different diameters. Most preferably, as may be seen from the cross sectional view provided in Figure 14, the second depth of the first cavity 2050 would have a cavity wherein a distal end portion 4010 of the first needle 4000 is extending. Preferably, the first circular recess 2050 is in fluid communication with a distal end of a first proximally extending piercing needle 4000. Similarly, the second circular recess 2054 is configured to be in fluid communication with a distal end of a second proximally positioned piercing needle 4050. Both the first and second needles 4000, 4050 are rigidly mounted within the inner body 2000. In this preferred arrangement, when mounted to the drug delivery device containing two medicament cartridges, the distal end of the first piercing needle 4000 will reside in fluid communication with the first cavity while the proximal piercing end of the first needle 4000 will reside in fluid communication with a medicament contained in a first cartridge within the drug delivery device. The second piercing needle 4050 will be configured in a similar fashion as the first piercing needle 4000, residing in fluid communication with the second cavity 2054 and a second medicament in a second cartridge.

In addition, and returning to the perspective view of inner body provided in Figure 18, the generally flat surface 2040 of the inner body further defines a holding chamber or a third cavity 2060. This third cavity 2060 is positioned near the distal end 2002 of the inner body 2000. As illustrated, this third cavity 2060 may have a generally rectangular shape. As described in greater detail below, this third cavity 2060 acts as a holding chamber for either a first fluid contained within a first cartridge, a second fluid contained within a second cartridge, or both fluids contained within a drug delivery device. Where either the first and/or second fluid comprises a medicament, this third cavity 2060 can act as a holding chamber during either a dose priming step and/or a dose administration step.

As can be seen from Figure 18, the inner body 2000 further comprises a distal neck portion 2080 and this distal neck portion comprises a generally flat surface 2082. This neck portion 2080 of the inner body 2000 has a generally circular shape defining a diameter Dnp 2086. The diameter Dnp 2086 of the neck portion 2080 is preferably sized to be of a similar dimension as the diameter of the outlet septum 2900 (see, e.g., Figure 14).

A recess 2044 is provided within this distal neck portion 2080 and this recess 2044 extends internally along the neck portion 2080 from the top flat surface 2082 towards the third cavity 2060. Most preferably, the recess 2044 defines an internal fluid channel that is configured to provide fluid communication with the third cavity 2060 provided along the flat surface 2040 of the inner body 2000. As such, when a dose dispenser, such as the double ended needle assembly, is connected to the dispense interface 1200, the proximally extending needle of the needle assembly will be guided into this recess 2044 by the needle guide 3000. Consequently, this proximal needle will reside in fluid communication with any fluid and/or medicament(s) residing in the third cavity or holding chamber 2060.

When the dispense interface 1210 is assembled, a pierceable septum 2900 will be positioned so as to reside along this top flat surface 2082. Then, the metallic ferrule 2800 will be provided over this distal end of the septum 2900 and then crimped over the septum 2900 and hence around the outer diameter Dnp 2086 of the neck portion 2080. As such, and as illustrated in the cross sectional view provided in Figures 14, the ferrule 2800 will act to hold the septum 2900 in place along the flat surface 2082 of the neck portion 2080.

As may be seen from the cross sectional view provided in Figure 14, the inner body 2000 further comprises a first and a second piercing needle or cannula 4000, 4050. Generally, the first and second needles may be used to provide fluid communication between a cartridge contained within the drug delivery device and either the first or second recess or reservoir 2050, 2054 of the inner body 2000.

In one preferred arrangement, both the first and the second needles 4000, 4050 are permanently affixed to the inner body 2000. The connection between the first and second needles 4000, 4050 and the inner body 2000 may be achieved through one of or a combination of an interference fit, a retaining adhesive, or any other suitable means for connected the needles to the inner body. In an example embodiment, a combination of an interference fit and a retaining adhesive is used. Figures 15 and 16 show a flat proximal surface 2066 of the inner body 2000. As may be seen from these figures, the flat proximal surface 2066 comprises first and second apertures 2070, 2074. With respect to the first aperture 2070, a first taper 2072 may be molded into the first aperture so as to provide an area for an adhesive to be provided when the first needle 4000 is inserted. In order to prevent the adhesive from coming into contact with and potentially contaminating medicament contained within or potentially flowing within the reservoir, the first aperture 2070 tapers to an interference fit. Such an interference fit can provide a seal between the reservoir and the adhesive applied within the taper 2072. The second needle 4050 may be attached to the second aperture 2074 of the inner body 2000 in a similar manner by way of the second taper 2076.

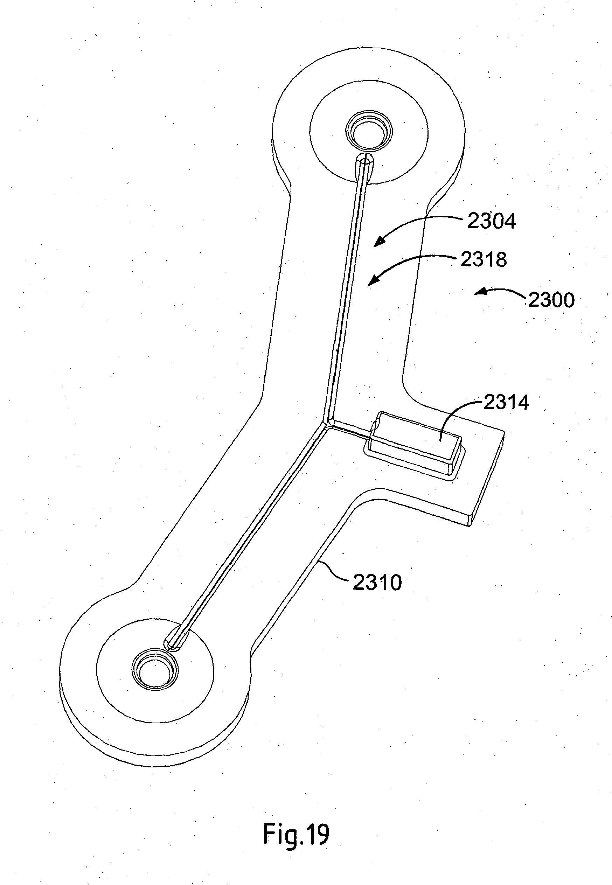

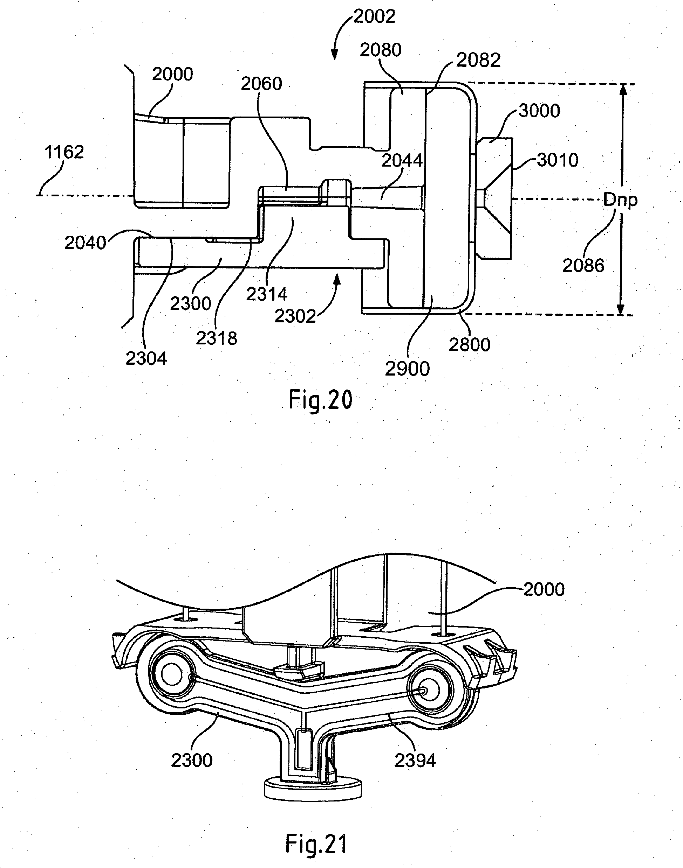

As may be seen from the partial cross sectional view illustrated in Figure 14 and the exploded views of the dispense interface 1200 illustrated in Figures 15 and 16, the dispense interface 1200 further comprises a manifold 2300. Preferably, this manifold 2300 comprises a shape that is generally similar to the Y-shape of the distal portion 2002 of the inner body 2000. For example, Figure 19 provides a perspective view of one arrangement of a manifold 2300 that may be used with the dispense interface 1210. As illustrated in Figures 15-16 and 19, the manifold 2300 comprises a generally flat top surface 2304 and a generally flat bottom surface 2310. In a preferred arrangement of the dispense interface 1210, the manifold bottom surface 2310 is positioned to reside along the generally flat surface 2040 of the inner body 2000. Preferably, in order to provide a seal between the manifold and the inner body 2000, these two components may be laser welded together. In order to facilitate such a laser welding seal, in one arrangement, the inner body 2000 may be molded of Cyclo Olefin Polymer ("COP") material that is preferably doped with a laser-welding additive. Such a laser welding additive may increase the inner body's sensitivity to laser light. In addition, the manifold 2300 may be molded in an optically clear COP so as to allow the welding laser to pass through the manifold 2300 and activate a mating surface residing between the two components with minimal interface. For example, Figure 21 illustrates the manifold 2300 provided along the flat surface of the inner body and then laser welded along a laser welding track 2394. As shown, this laser welding track 2394 extends along an outer edge of the manifold 2300. The large, flat mating surface areas 2304, on the manifold and the inner body 2040 respectively, help to produce a substantial surface area for the welding to act upon and this tends to maximize the seal created between these two components.
Preferably, the manifold 2300 further comprises a fluid groove arrangement 2318 and a rectangular protrusion or filling block 2314. For example, Figure 20 illustrates a partial sectional view of the manifold 2300 laser welded to the inner body 2000. As illustrated, referring to Figures 19 and 20, both the groove arrangement 2318 and the protrusion or filling block 2314 may be provided along a manifold top surface 2304. The protrusion 2314 may be provided near a distal end 2302 of the manifold 2300. In one preferred arrangement, this protrusion 2314 comprises a rectangular protrusion. With such a rectangular configuration, once the manifold 2300 is assembled (e.g., laser welded) along the flat surface 2040 of the inner body 2000, the protrusion 2314 will reside within the third cavity or holding chamber 2060 of the inner body 2000. As illustrated, the rectangular protrusion or the filling block fills the majority of the third cavity or holding chamber while still redirecting fluid flow. One advantage of such a configuration is that it reduces the ullage of the dispense interface 1200.

In addition, forming the fluid groove arrangement 2318 as a cavity between the two laser welded components allows the majority of the fluid groove geometry to be molded using an open-and-shut tool. Consequently, use of an open-and-shut tool reduces the need for fragile core pins or split lines with the fluid groove arrangement. This also allows for relatively complex and tight tolerance geometry without complex tooling. The molding of key assembly snap features on the same component, such as the outer protrusion 2006 on the inner body 2000, also helps reduce tolerance stack-ups and also tends to allow for small needle wells and therefore smaller ullage.

In addition, the use of the needle guide 3000 to direct a Type A cannula means that the channel into which the cannula is received can be smaller as some of the tolerances on the needle position are reduced. The alignment of the flow path through the dispense interface also requires certain special considerations. As just one example, in one example arrangement, both of the cartridges contained within the drug delivery device as well as the needle assembly are positioned in a single plane cutting through the depth of the drug delivery device along the device centerline 1162. However, due to the positioning of the diaphragm valves 2700, 2750 and the fluid groove arrangement 2318 on one side of the dispense interface components, the fluid groove arrangement 2318 is moved off this centerline 1162. Prior to dispense through an attached needle assembly, this groove arrangement 2318 is brought back onto the centerline 1162 using the third cavity or holding chamber 2060 molded into the inner body 2000. These factors combine to reduce the volume of liquid or medicament required to fill the dispense interface 1200 prior to dispense, thereby aiding dose accuracy.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin. Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω)-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39),
   wherein the group -Lys6-NH2 may be bound to the C-terminus of the Exendin-4 derivative;
or an Exendin-4 derivative of the sequence
   H-(Lys)6-des Pro36 [Asp28] Exendin-4(1-39)-Lys6-NH2,
   des Asp28 Pro36, Pro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2,
   H-Asn-(Glu)5-des Pro36, Pro37, Pro38 [Asp28] Exendin-4(1-39)-(Lys)6-NH2,
   H-(Lys)6-des Pro36 [Trp(O2)25, Asp28] Exendin-4(1-39)-Lys6-NH2,
   H-des Asp28 Pro36, Pro37, Pro38, [Trp(O2)25] Exendin-4(1-39)-NH2,
   H-(Lys)6-des Pro36, Pro37, Pro38 [Trp(O2)25, Asp28] Exendin-4(1-39)-NH2,
   H-Asn-(Glu)5-des Pro36, Pro37, Pro38 [Trp(O2)25, Asp28] Exendin-4(1-39)-NH2,
   des Pro36, Pro37, Pro38 [Trp(O2)25, Asp28] Exendin-4(1-39)-(Lys)6-NH2,
   H-(Lys)6-des Pro36, Pro37, Pro38 [Trp(O2)25, Asp28] Exendin-4(1-39)-(Lys)6-NH2,
   H-Asn-(Glu)5-des Pro36, Pro37, Pro38 [Trp(O2)25, Asp28] Exendin-4(1-39)-(Lys)6-NH2,
   H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2,
   H-Asn-(Glu)5-des Pro36, Pro37, Pro38 [Met(O)14, Asp28] Exendin-4(1-39)-NH2,
   des Pro36, Pro37, Pro38 [Met(O)14, Asp28] Exendin-4(1-39)-(Lys)6-NH2,
   H-(Lys)6-des Pro36, Pro37, Pro38 [Met(O)14, Asp28] Exendin-4(1-39)-(Lys)6-NH2,
   H-Asn-(Glu)5 des Pro36, Pro37, Pro38 [Met(O)14, Asp28] Exendin-4(1-39)-(Lys)6-NH2,
   H-Lys6-des Pro36 [Met(O)14, Trp(O2)25, Asp28] Exendin-4(1-39)-Lys6-NH2,
   H-des Asp28 Pro36, Pro37, Pro38 [Met(O)14, Trp(O2)25] Exendin-4(1-39)-NH2,
   H-(Lys)6-des Pro36, Pro37, Pro38 [Met(O)14, Asp28] Exendin-4(1-39)-NH2,
   H-Asn-(Glu)5-des Pro36, Pro37, Pro38 [Met(O)14, Trp(O2)25, Asp28] Exendin-4(1-39)-NH2,
   des Pro36, Pro37, Pro38 [Met(O)14, Trp(O2)25, Asp28] Exendin-4(1-39)-(Lys)6-NH2,
   H-(Lys)6-des Pro36, Pro37, Pro38 [Met(O)14, Trp(O2)25, Asp28] Exendin-4(S1-39)-(Lys)6-NH2,
   H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin odium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by A and κ. A light chain has two successive domains: one constant domain (CL) and one variable domain (VL). The approximate length of a light chain is 211 to 217 amino acids. Each antibody contains two light chains that are always identical; only one type of light chain, κ or A, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

## Claims

1. Apparatus comprising:
- an inner body (2000) of a dispense interface and
- a manifold (2300),
wherein the inner body (2000) comprises:
- a recess (2044) configured to receive a distal needle from a distal end (2002) of the inner body (2000); and
- a first fluid reservoir (2050) and a second fluid reservoir (2054);
wherein the inner body (2000) and the manifold (2300) are assembled such that there is formed a channel providing a fluid connection from the first fluid reservoir (2050) and the second fluid reservoir (2054), respectively, to the recess (2044);
**characterized in**
**that** the manifold (2300) comprises:
- a filling block (2314) configured to redirect flow;
- a fluid groove arrangement (2318) configured to provide a fluid connection to the filling block (2314);
and **that**
the fluid connection from the first fluid reservoir (2050) and the second fluid reservoir (2054), respectively, to the recess (2044) is provided via the fluid groove arrangement (2318) and the filling block (2314).

2. Apparatus according to claim 1, wherein the inner body (2000) comprises a cavity (2060) with a fluid connection to the recess (2044), which cavity (2060) is arranged on an axis defined by the recess (2044) and wherein the filling block (2314) comprises a protrusion, and wherein the inner body (2000) and the manifold (2300) are assembled such that the cavity (2600) receives the filling block (2314).

3. Apparatus according to claim 2, wherein the protrusion is configured to fill more than half of a volume of the cavity (2060).

4. Apparatus according to one of claims 1 to 3, wherein the inner body (2000) comprises at least one outwardly protruding member (2006, 2014) configured to form an interference fit with an outer body of the dispense interface.

5. Apparatus according to one of claims 1 to 4, wherein the inner body (2000) comprises a flat surface (2040) and wherein the recess (2044) is arranged on a plane of symmetry of the flat surface (2040), which plane of symmetry is parallel to a longitudinal axis of the inner body (2000).

6. Apparatus according to claim 5, wherein the first fluid reservoir (2050) and the second fluid reservoir (2054) are arranged symmetrically with respect to the plane of symmetry of the flat surface (2040).

7. Apparatus according to claim 5 or 6, wherein the channel is formed at a boundary between the manifold (2300) and the inner body (2000).

8. Apparatus according to one of claims 5 to 7, wherein the inner body (2000) and the manifold (2300) are assembled by laser-welding.

9. Apparatus according to one of claims 5 to 8, wherein the inner body (2000) comprises a first aperture (2070) and a second aperture (2074) each configured to receive a first proximal needle and a second proximal needle, respectively, for providing a respective fluid connection to the first fluid reservoir (2050) and the second fluid reservoir (2054).

10. Apparatus according to claim 9, wherein the first aperture (2070) and the second aperture (2074) are arranged symmetrically with respect to the plane of symmetry of the flat surface (2040).

11. Apparatus according to one of claims 1 to 10 comprising a needle guide (3000) arranged at a distal end (2002) of the inner body (2000), which needle guide (3000) comprises a needle recess (3010).

12. Apparatus according to claim 11, wherein the needle guide (3000) is configured to align a distal needle to the recess (2044).

13. Apparatus according to claim 11 or 12 comprising a pierceable septum (2900) arranged along a flat surface (2082) of a distal neck portion (2080) of the inner body (2000) and configured to receive a needle passing the needle guide (3000).

14. Apparatus according to claim 13 comprising a ferrule (2800) arranged over the septum (2900) and over an outer diameter of the distal neck portion (2080) of the inner body (2000).

15. Drug delivery device comprising an apparatus according to one of claims 1 to 14.

## Patentansprüche

1. Vorrichtung umfassend:
- einen Innenkörper (2000) einer Ausgabeschnittstelle und
- eine Sammeleinrichtung (2300),
wobei der Innenkörper (2000) umfasst:
- eine Ausnehmung (2044), die zum Aufnehmen einer distalen Nadel von einem distalen Ende (2002) des Innenkörpers (2000) konfiguriert ist; und
- ein erstes Fluidreservoir (2050) und ein zweites Fluidreservoir (2054);
wobei der Innenkörper (2000) und die Sammeleinrichtung (2300) derart zusammengefügt sind, dass ein Kanal ausgebildet wird, der eine Fluidverbindung von dem ersten Fluidreservoir (250), beziehungsweise dem zweiten Fluidreservoir (2054) mit der Vertiefung (2044) vorsieht,
**dadurch gekennzeichnet, dass**
die Sammeleinrichtung (2300) umfasst:
- einen Füllblock (2314), der zum Umleiten des Flusses konfiguriert ist;
- eine Fluidrinnenanordnung (2318), die zum Bereitstellen einer Fluidverbindung mit dem Füllblock (2314) konfiguriert ist;
und dass
die Fluidverbindung von dem ersten Fluidreservoir (250), beziehungsweise dem zweiten Fluidreservoir (2054) mit der Ausnehmung (2044) durch die Fluidrinnenanordnung (2318) und den Füllblock (2314) bereitgestellt wird.

2. Vorrichtung nach Anspruch 1, wobei der Innenkörper (2000) einen Hohlraum (2060) mit einer Fluidverbindung zu der Ausnehmung (2044) aufweist, wobei der Hohlraum (2060) an einer durch die Ausnehmung (2044) festgelegten Achse angeordnet ist und wobei der Füllblock (2314) einen Vorsprung aufweist und wobei der Innenkörper (2000) und die Sammeleinrichtung (2300) derart zusammengefügt sind, dass der Hohlraum (2600) den Füllblock (2314) aufnimmt.

3. Vorrichtung nach Anspruch 2, wobei der Vorsprung konfiguriert ist, mehr als die Hälfte eines Volumens von dem Hohlraum (2060) zu füllen.

4. Vorrichtung nach einem der Ansprüche 1 bis 3, wobei der Innenkörper (2000) mindestens ein nach außen vorstehendes Element (2006, 2014) aufweist, das konfiguriert ist, eine Presspassung mit einem Außenkörper der Ausgabeschnittstelle auszubilden.

5. Vorrichtung nach einem der Ansprüche 1 bis 4, wobei der Innenkörper (2000) eine ebene Fläche (2040) aufweist und wobei die Ausnehmung (2044) auf einer Symmetrieebene der ebenen Fläche (2040) angeordnet ist, wobei die Symmetrieebene parallel zu einer Längsachse des Innenkörpers (2000) liegt.

6. Vorrichtung nach Anspruch 5, wobei das erste Fluidreservoir (2050) und das zweite Fluidreservoir (2054) symmetrisch bezüglich der Symmetrieebene der ebenen Fläche (2040) angeordnet sind.

7. Vorrichtung nach Anspruch 5 oder 6, wobei der Kanal an einer Grenze zwischen der Sammeleinrichtung (2300) und dem Innenkörpers (2000) ausgebildet wird.

8. Vorrichtung nach einem der Ansprüche 5 bis 7, wobei der Innenkörper (2000) und die Sammeleinrichtung (2300) mittels Laserschweißen zusammengefügt sind.

9. Vorrichtung nach einem der Ansprüche 5 bis 8, wobei der Innenkörper (2000) eine erste Öffnung (2070) und eine zweite Öffnung (2074), die jeweils konfiguriert sind, um eine erste proximale Nadel beziehungsweise eine zweite proximale Nadel aufzunehmen, zum Bereitstellen einer entsprechenden Fluidverbindung mit dem ersten Fluidreservoir (2050) und dem zweiten Fluidreservoir (2054) aufweist.

10. Vorrichtung nach Anspruch 9, wobei die erste Öffnung (2070) und die zweite Öffnung (2074) symmetrisch bezüglich der Symmetrieebene der ebenen Fläche (2040) angeordnet sind.

11. Vorrichtung nach einem der Ansprüche 1 bis 10, umfassend eine Nadelführung (3000), die an einem distalen Ende (2002) des Innenkörpers (2000) angeordnet ist, wobei die Nadelführung (3000) eine Nadelvertiefung (3010) aufweist.

12. Vorrichtung nach Anspruch 11, wobei die Nadelführung (3000) konfiguriert ist, eine distale Nadel mit der Ausnehmung (2044) auszurichten.

13. Vorrichtung nach Anspruch 11 oder 12, die ein durchstechbares Septum (2900) aufweist, das entlang einer ebenen Fläche (2082) eines distalen Ansatzbereichs (2080) des Innenkörpers (2000) angeordnet und konfiguriert ist, eine Nadel aufzunehmen, welche durch die Nadelführung (3000) hindurchgeht.

14. Vorrichtung nach Anspruch 13, die eine Presshülse (2800) aufweist, welche über dem Septum (2900) und über einem Außendurchmesser des distalen Ansatzbereichs (2080) des Innenkörpers (2000) angeordnet ist.

15. Arzneimittelabgabevorrichtung, umfassend eine Vorrichtung nach einem der Ansprüche 1 bis 14.

## Revendications

1. Appareil, comprenant:
un corps intérieur (2000) d'une interface d'administration, et
un collecteur (2300),
dans lequel le corps intérieur (2000) comprend:
un renfoncement (2044) configuré pour recevoir une aiguille distale à partir d'une extrémité distale (2002) du corps intérieur (2000), et
un premier réservoir de fluide (2050) et un deuxième réservoir de fluide (2054),
dans lequel le corps intérieur (2000) et le collecteur (2300) sont assemblés de telle sorte qu'un canal soit formé qui établit une connexion fluidique partant du premier réservoir de fluide (2050) et du deuxième réservoir de fluide (2054), respectivement, jusqu'au renfoncement (2044),
**caractérisé en ce que**:
le collecteur (2300) comprend:
un bloc de remplissage (2314) configuré pour rediriger un écoulement,
un agencement de rainure de fluide (2318) configuré pour établir une connexion fluidique jusqu'au bloc de remplissage (2314),
et **en ce que** la connexion fluidique partant du premier réservoir de fluide (2050) et du deuxième réservoir de fluide (2054), respectivement, jusqu'au renfoncement (2044) est réalisée par l'intermédiaire de l'agencement de rainure de fluide (2318) et du bloc de remplissage (2314).

2. Appareil selon la revendication 1, dans lequel le corps intérieur (2000) comprend une cavité (2060) présentant une connexion fluidique avec le renfoncement (2044), ladite cavité (2060) étant agencée sur un axe défini par le renfoncement (2044), et dans lequel le bloc de remplissage (2314) comporte une saillie, et dans lequel le corps intérieur (2000) et le collecteur (2300) sont assemblés de telle sorte que la cavité (2600) reçoive le bloc de remplissage (2314).

3. Appareil selon la revendication 2, dans lequel la saillie est configurée pour remplir plus de la moitié d'un volume de la cavité (2060).

4. Appareil selon l'une quelconque des revendications 1 à 3, dans lequel le corps intérieur (2000) comprend au moins un élément saillant vers l'extérieur (2006, 2014) configuré pour former un ajustement serré avec un corps extérieur de l'interface d'administration.

5. Appareil selon l'une quelconque des revendications 1 à 4, dans lequel le corps intérieur (2000) présente une surface plate (2040), et dans lequel le renfoncement (2044) est situé sur un plan de symétrie de la surface plate (2040), ledit plan de symétrie étant parallèle à un axe longitudinal du corps intérieur (2000).

6. Appareil selon la revendication 5, dans lequel le premier réservoir de fluide (2050) et le deuxième réservoir de fluide (2054) sont agencés de façon symétrique par rapport au plan de symétrie de la surface plate (2040).

7. Appareil selon la revendication 5 ou 6, dans lequel le canal est formé à une frontière entre le collecteur (2300) et le corps intérieur (2000).

8. Appareil selon l'une quelconque des revendications 5 à 7, dans lequel le corps intérieur (2000) et le collecteur (2300) sont assemblés par soudage au laser.

9. Appareil selon l'une quelconque des revendications 5 à 8, dans lequel le corps intérieur (2000) comporte une première ouverture (2070) et une deuxième ouverture (2074) configurées chacune pour recevoir une première aiguille proximale et une deuxième aiguille proximale, respectivement, afin de fournir une connexion fluidique respective au premier réservoir de fluide (2050) et au deuxième réservoir de fluide (2054).

10. Appareil selon la revendication 9, dans lequel la première ouverture (2070) et la deuxième ouverture (2074) sont agencées de façon symétrique par rapport au plan de symétrie de la surface plate (2040).

11. Appareil selon l'une quelconque des revendications 1 à 10, comprenant un guide d'aiguille (3000) qui est agencé à une extrémité distale (2002) du corps intérieur (2000), ledit guide d'aiguille (3000) comprenant un renfoncement d'aiguille (3010).

12. Appareil selon la revendication 11, dans lequel le guide d'aiguille (3000) est configuré pour aligner une aiguille distale avec le renfoncement (2044).

13. Appareil selon la revendication 11 ou 12, comprenant un septum à percer (2900) qui est agencé le long d'une surface plate (2082) d'une partie de col distale (2080) du corps intérieur (2000) et qui est configuré pour recevoir une aiguille passant par le guide d'aiguille (3000).

14. Appareil selon la revendication 13, comprenant une virole (2800) qui est agencée au-dessus du septum (2900) et sur un diamètre extérieur de la partie de col distale (2080) du corps intérieur (2000).

15. Dispositif d'administration de médicament comprenant un appareil selon l'une quelconque des revendications 1 à 14.
